# EUROPEAN PATENT APPLICATION

(11) **EP 4 299 737 A1**
(43) Date of publication of application: **03.01.2024**
(21) Application number: 22781636.0
(22) Date of filing: 31.03.2022
(51) Int. Cl.: C12N 11/087, C12N 11/04, C12N 5/071, C12N 5/00

(54) **TISSUE-DERIVED EXTRACELLULAR MATRIX DERIVATIVE MODIFIED WITH ACRYL GROUP AND USE THEREOF**

(30) Priority: 31.03.2021 KR 20210042153
(71) Applicant: Cellartgen Inc., Seoul 03722 (KR)
(72) Inventor: CHO, Seung Woo, Seoul 03722 (KR); AN, Soo Hwan, Namyangju-si Gyeonggi-do 12219 (KR); KIM, Su Kyeom, Seoul 04208 (KR); KIM, Yu Heun, Seoul 03726 (KR)
(74) Representative: Manitz Finsterwald Patent- und Rechtsanwaltspartnerschaft mbB
(86) International application number: PCT/KR2022/004584
(87) International publication number: WO 2022/211517

(57) **Abstract**

The present invention relates to a tissue-derived extracellular matrix derivative modified with an acryl group and a use thereof. A composition for a hydrogel and a hydrogel prepared therefrom of the present invention can simulate fibrosis patterns of ^{A} various tissues according to the degree of modification or crosslinking.

## Description

### TECHNICAL FIELD

The present disclosure relates to a tissue-derived extracellular matrix derivative modified with an acryl group and a use thereof.

### BACKGROUND

Fibrosis of tissue is caused by the excessive production and accumulation of an extracellular matrix, which is mainly collagen, in tissue. When tissue is damaged by a stimulus such as oxidative stress, hypoxia, inflammation, or apoptosis, damaged tissue is repaired by replacement with an extracellular matrix, but in the case of the damage being serious or in the case of such stimulation becoming chronic, the accumulation of an extracellular matrix becomes excessive, and the tissue cannot perform its function sufficiently. Fibrosis is seen in various types of organs, such as the liver, pancreas, lung, kidney, bone marrow, and heart, and it is thought that collagen-producing cells such as myofibroblasts are related to a disease state.

Liver fibrosis is a disease caused by the abnormal accumulation of an extracellular matrix (ECM) and can progress to cirrhosis or liver cancer. When liver fibrosis occurs, chemokines released by damaged liver cells or vascular cells attract macrophages and hepatic stellate cells present in liver tissue due to the secreted TGF-β become myofibroblast-like cells to produce the ECM. However, not much is known about substances or methods for preventing and treating liver fibrosis.

Also, kidney fibrosis refers to a symptom in which the tissues and/or blood vessels of the kidney are hardened, and lung fibrosis or fibroid lung is known as a disease characterized by diffuse fibroplasia in the alveolar wall, with major symptoms, such as a dry cough or shortness of breath during working.

Accordingly, the present inventors developed a model that can be used to study fibrosis and achieved the present disclosure.

### DISCLOSURE OF THE INVENTION

### PROBLEMS TO BE SOLVED BY THE INVENTION

The present disclosure is conceived to provide a composition for hydrogel, including a tissue-derived extracellular matrix modified with an acryl group.

The present disclosure is also conceived to provide a hydrogel prepared by crosslinking the composition.

The present disclosure is also conceived to provide a method of preparing a composition for hydrogel, including a process of modifying a tissue-derived extracellular matrix with an acryl group.

The present disclosure is also conceived to provide a method of preparing a hydrogel, including: a process of preparing a composition for hydrogel by modifying a tissue-derived extracellular matrix with an acryl group; and a process of crosslinking the composition for hydrogel.

### MEANS FOR SOLVING THE PROBLEMS

In order to solve the above-described problems, an aspect of the present disclosure provides a composition for hydrogel, including a tissue-derived extracellular matrix modified with an acryl group.

In an embodiment of the present disclosure, the acryl group may be any one or more of an acrylate group, a methacrylate group, and an itaconate group.

In an embodiment of the present disclosure, the tissue-derived extracellular matrix may be any one or more of a liver tissue-derived extracellular matrix, a lung tissue-derived extracellular matrix, a kidney tissue-derived extracellular matrix, a heart tissue-derived extracellular matrix, an intestinal tissue-derived extracellular matrix, a muscle tissue-derived extracellular matrix, a skin tissue-derived extracellular matrix, a pancreatic tissue-derived extracellular matrix, a bone marrow tissue-derived extracellular matrix, a brain tissue-derived extracellular matrix, and a spinal cord tissue-derived extracellular matrix.

In an embodiment of the present disclosure, the weight ratio of the acryl group and the tissue-derived extracellular matrix may be 1:1 to 8:1.

In an embodiment of the present disclosure, the modification may be performed by a treatment at a pH of 8 to 14 for more than 0 hours to less than 8 hours, followed by a treatment at a pH of 6 to 8 for 12 hours to 28 hours.

In an embodiment of the present disclosure, the modification of the tissue-derived extracellular matrix with the acryl group may be performed in a range of 50% to 75%.

Another aspect of the present disclosure provides a hydrogel prepared by crosslinking the composition.

In an embodiment of the present disclosure, the composition may have a concentration of 0.1% (w/v) to 4.0% (w/v).

In an embodiment of the present disclosure, the composition may further include a photoinitiator, and the crosslinking may further include UV irradiation.

In an embodiment of the present disclosure, the UV irradiation may be performed for 1 minute to 20 minutes.

Yet another aspect of the present disclosure provides a method of preparing a composition for hydrogel, including a process of modifying a tissue-derived extracellular matrix with an acryl group.

In an embodiment of the present disclosure, the weight ratio of the tissue-derived extracellular matrix and the acryl group may be 1:1 to 1:8.

In an embodiment of the present disclosure, the modification may be performed by a treatment at a pH of 8 to 14 for more than 0 hours to less than 8 hours, followed by a treatment at a pH of 6 to 8 for 12 hours to 28 hours.

Still another aspect of the present disclosure provides a method of preparing a hydrogel, including: a process of preparing a composition for hydrogel by modifying a tissue-derived extracellular matrix with an acryl group; and a process of crosslinking the composition for hydrogel.

In an embodiment of the present disclosure, the composition for hydrogel may further include a photoinitiator, and the crosslinking may further include UV irradiation.

### EFFECTS OF THE INVENTION

A composition for hydrogel of the present disclosure and a hydrogel prepared therefrom can simulate fibrosis patterns of various tissues according to the degree of modification or crosslinking.

### BRIEF DESCRIPTION OF THE DRAWINGS

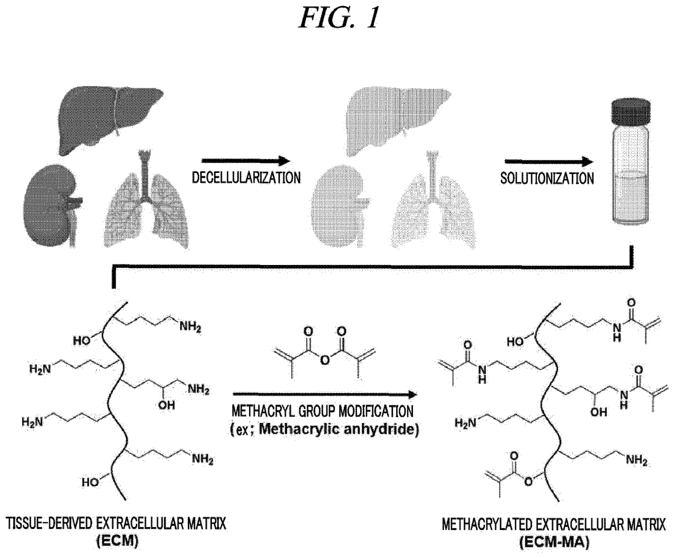

**FIG.** 1 is a schematic diagram showing the preparation of an ECM-MA derivative.

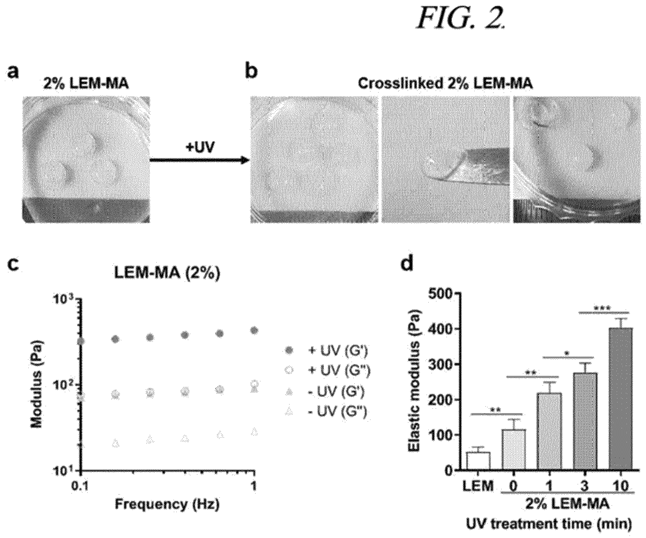

**FIG. 2** shows the result confirming the possibility of preparing a hydrogel with the ECM-MA derivative and the possibility of controlling the physical properties by photo-crosslinking.

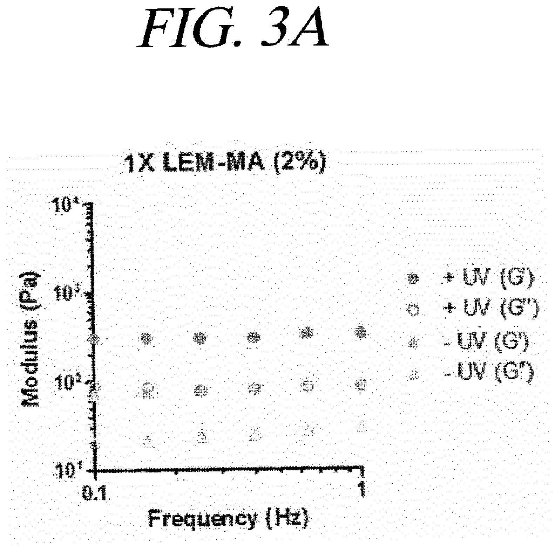

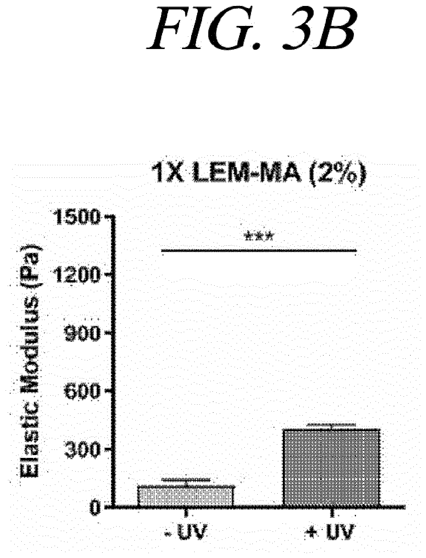

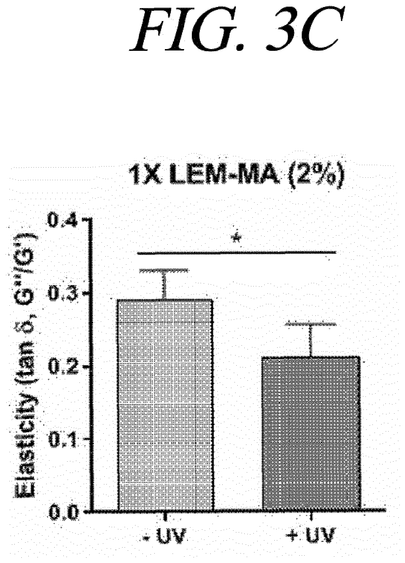

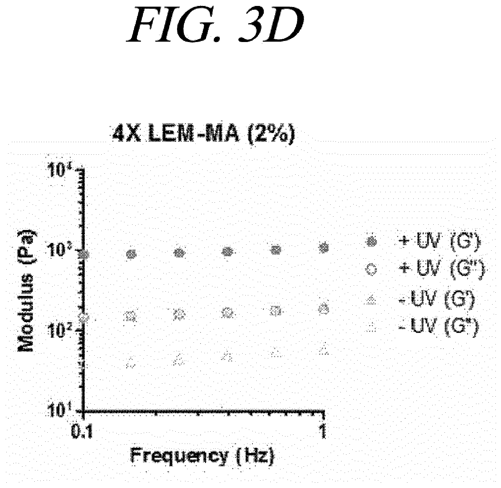

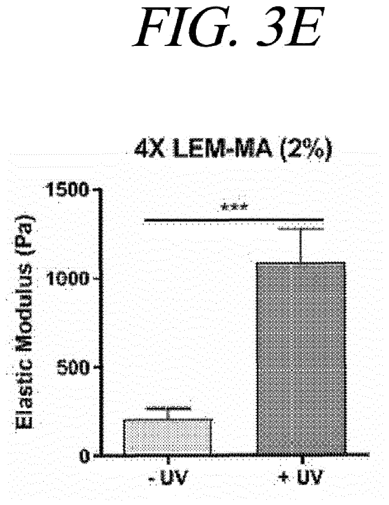

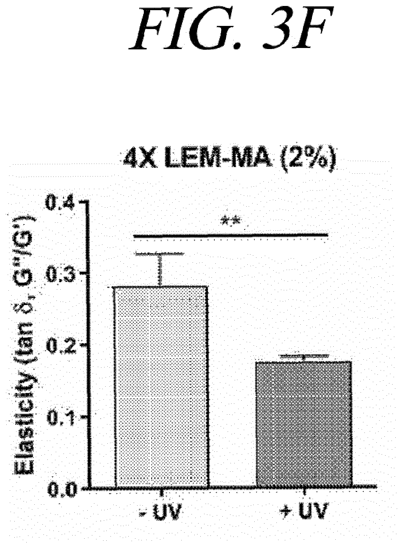

**FIG. 3A** shows the result confirming the possibility of controlling the mechanical properties by adjusting the modification ratio of a methacryl group in the ECM-MA derivative. **FIG. 3B** shows the result confirming the possibility of controlling the mechanical properties by adjusting the modification ratio of a methacryl group in the ECM-MA derivative. **FIG. 3C** shows the result confirming the possibility of controlling the mechanical properties by adjusting the modification ratio of a methacryl group in the ECM-MA derivative. **FIG. 3D** shows the result confirming the possibility of controlling the mechanical properties by adjusting the modification ratio of a methacryl group in the ECM-MA derivative. **FIG. 3E** shows the result confirming the possibility of controlling the mechanical properties by adjusting the modification ratio of a methacryl group in the ECM-MA derivative. **FIG. 3F** shows the result confirming the possibility of controlling the mechanical properties by adjusting the modification ratio of a methacryl group in the ECM-MA derivative.

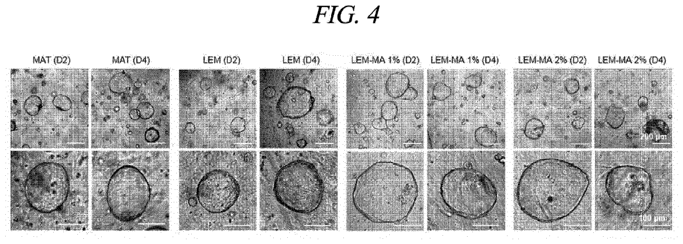

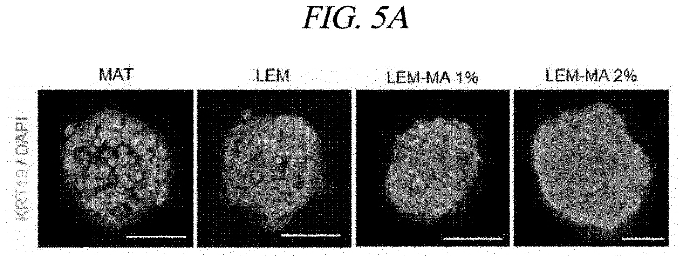

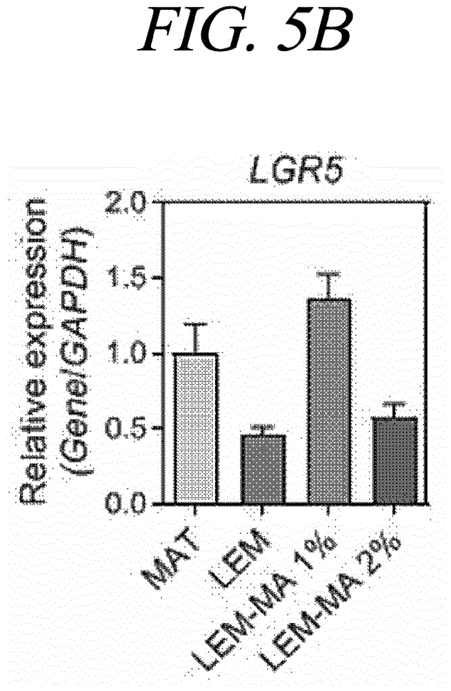

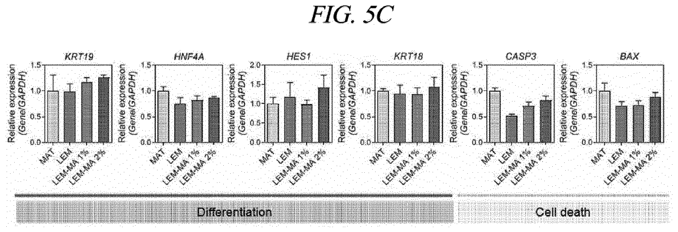

**FIG. 4** shows the result confirming the possibility of culturing mouse adult stem cell-derived liver organoids using an ECM-MA hydrogel. **FIG.** 5A shows the result confirming the possibility of culturing mouse adult stem cell-derived liver organoids using the ECM-MA hydrogel. FIG. 5B is a graph of the result confirming the possibility of culturing mouse adult stem cell-derived liver organoids using the ECM-MA hydrogel. FIG. 5C is a graph of the result confirming the possibility of culturing mouse adult stem cell-derived liver organoids using the ECM-MA hydrogel.

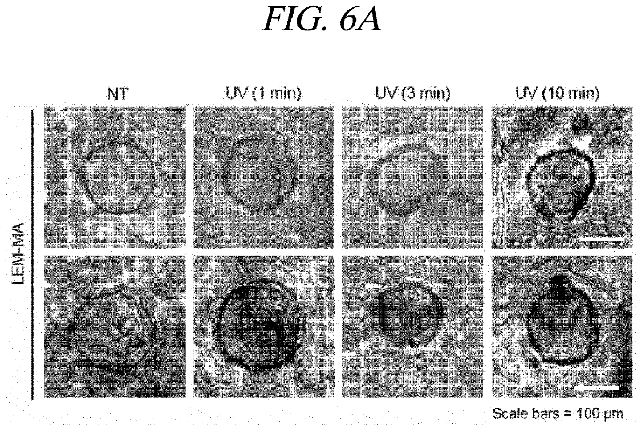

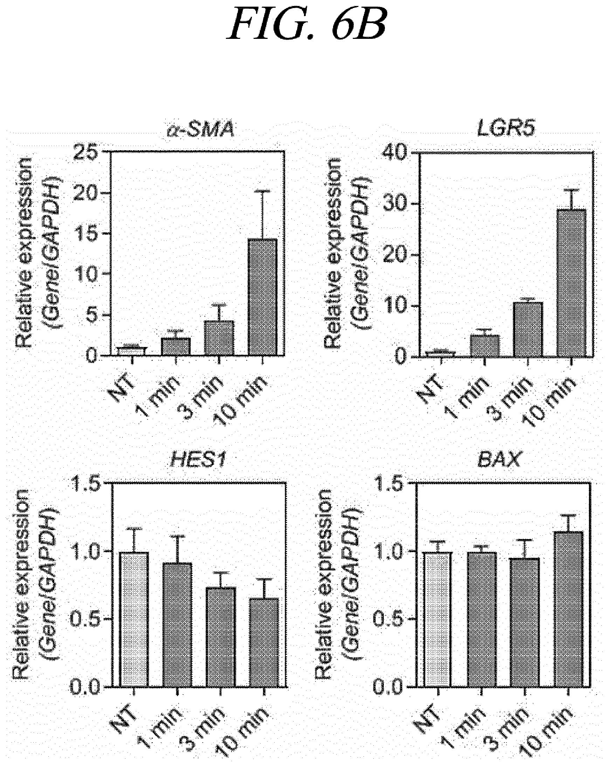

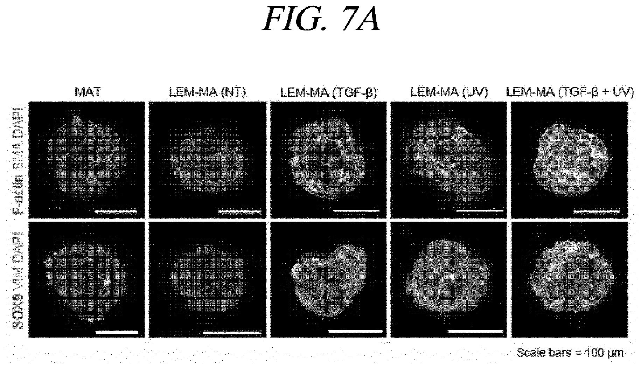

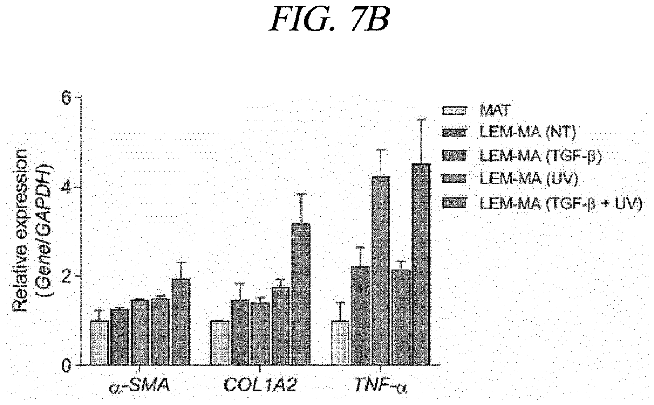

**FIG. 6A** shows the result of modeling mouse adult stem cell-derived liver organoid fibrosis using the ECM-MA hydrogel. FIG. 6B shows the result of modeling mouse adult stem cell-derived liver organoid fibrosis using the ECM-MA hydrogel. FIG. 7A shows the result of modeling mouse adult stem cell-derived liver organoid fibrosis using the ECM-MA hydrogel. **FIG. 7B** shows the result of modeling mouse adult stem cell-derived liver organoid fibrosis using the ECM-MA hydrogel.

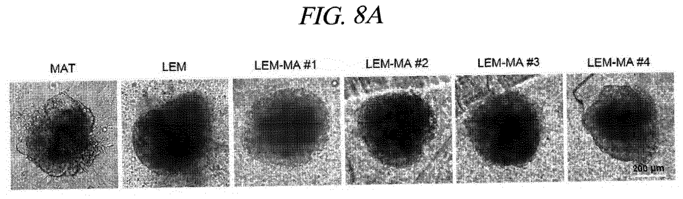

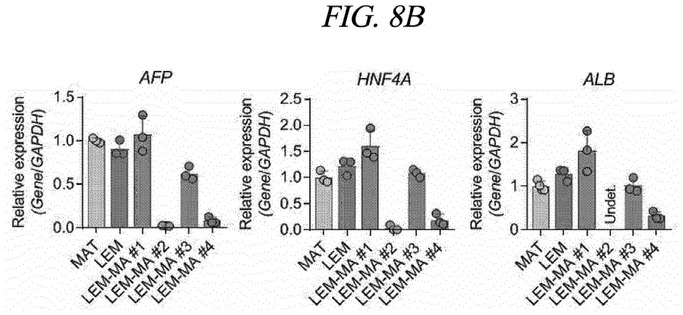

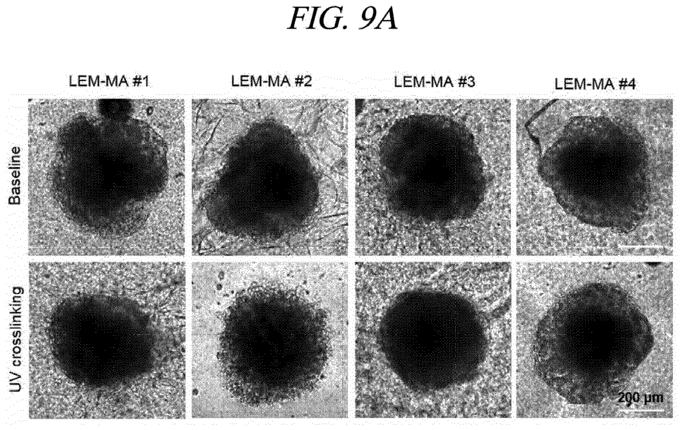

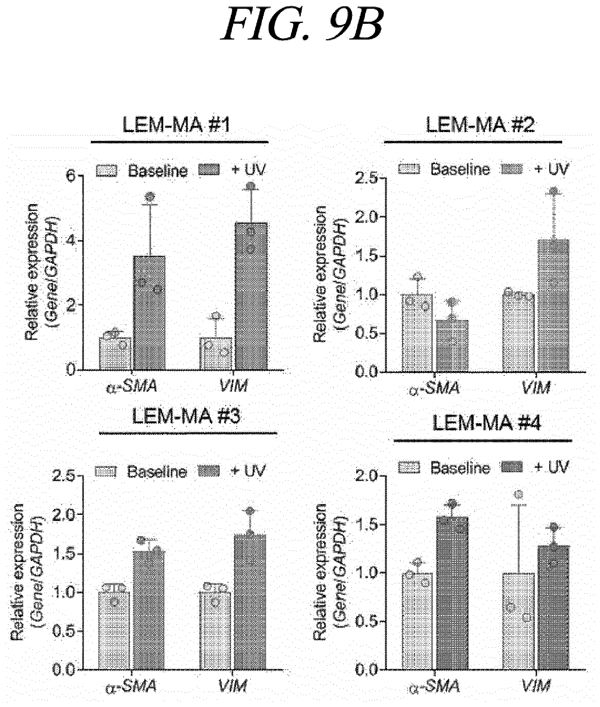

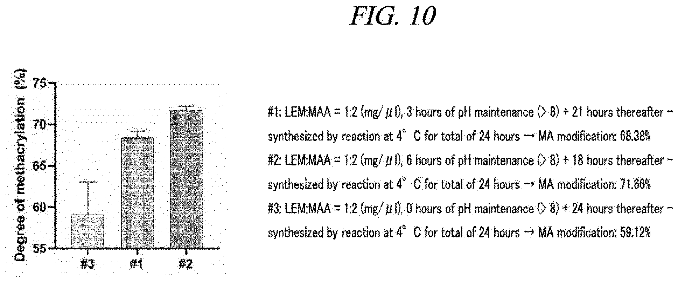

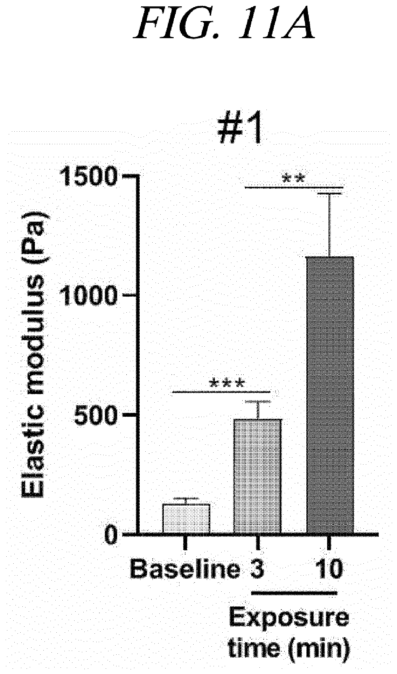

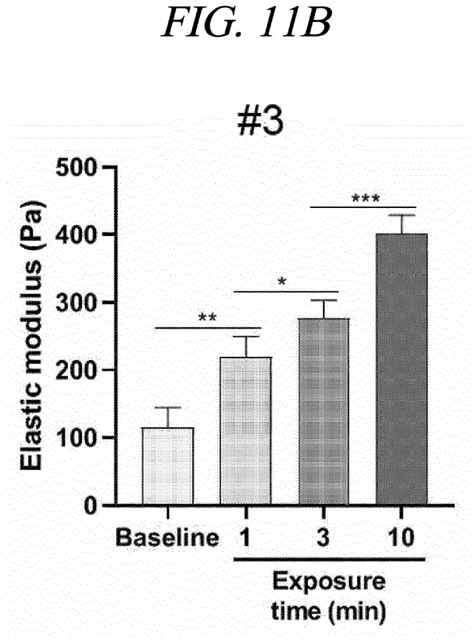

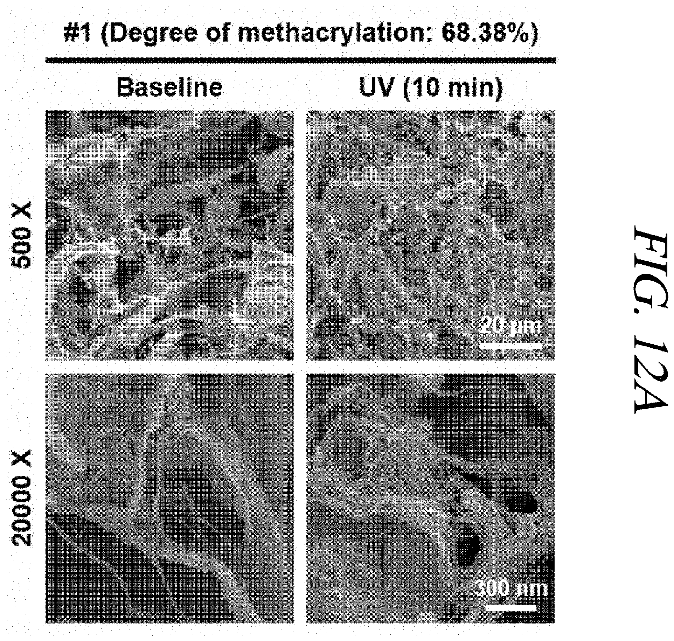

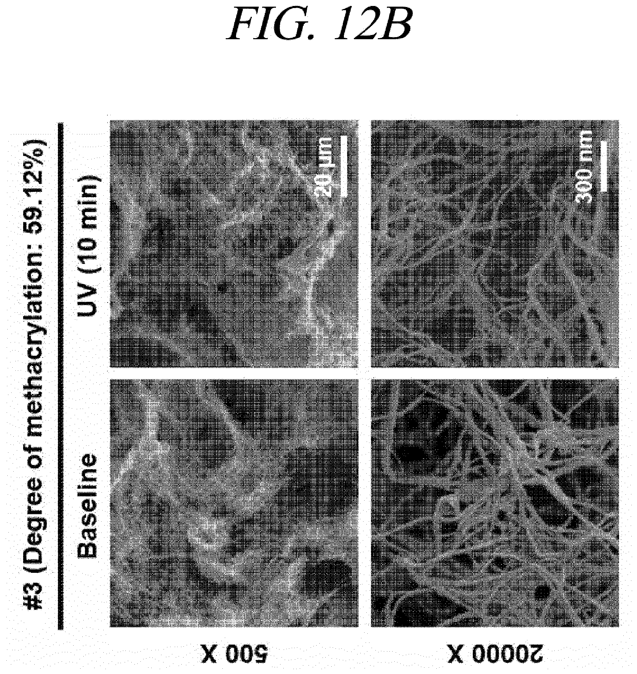

**FIG. 8A** shows an optimization result for modeling human iPSC-derived liver organoid fibrosis. **FIG. 8B** shows an optimization result for modeling human iPSC-derived liver organoid fibrosis. **FIG. 9A** shows an optimization result for modeling human iPSC-derived liver organoid fibrosis. **FIG. 9B** shows an optimization result for modeling human iPSC-derived liver organoid fibrosis. **FIG. 10** shows an optimization result for modeling human iPSC-derived liver organoid fibrosis. **FIG. 11A** shows an optimization result for modeling human iPSC-derived liver organoid fibrosis. FIG. 11B shows an optimization result for modeling human iPSC-derived liver organoid fibrosis. **FIG. 12A** shows an optimization result for modeling human iPSC-derived liver organoid fibrosis. **FIG. 12B** shows an optimization result for modeling human iPSC-derived liver organoid fibrosis.

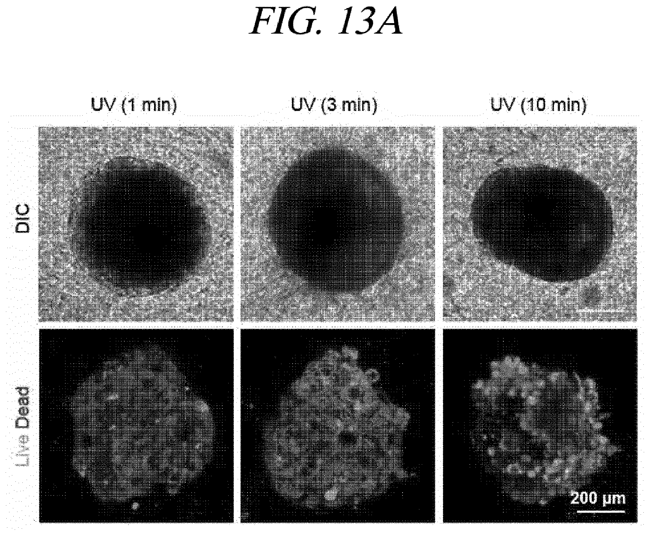

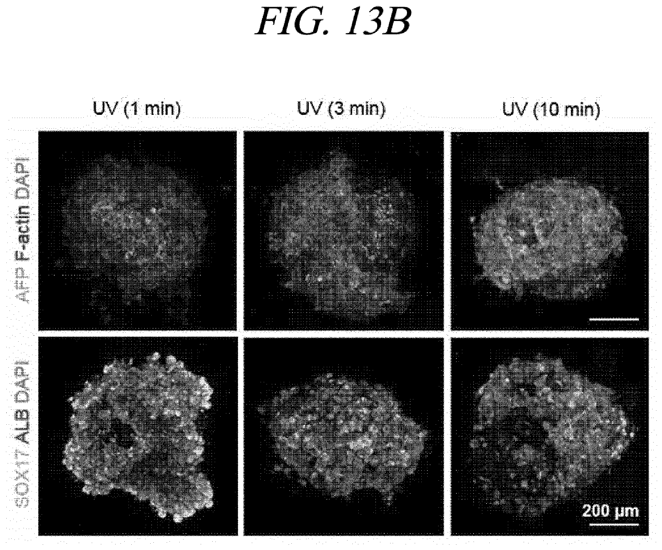

**FIG. 13A** shows the result of evaluation of toxicity of human iPSC-derived liver organoids depending on the photo-crosslinking time based on the ECM-MA hydrogel. **FIG. 13B** shows the result of evaluation of toxicity of human iPSC-derived liver organoids depending on the photo-crosslinking time based on the ECM-MA hydrogel.

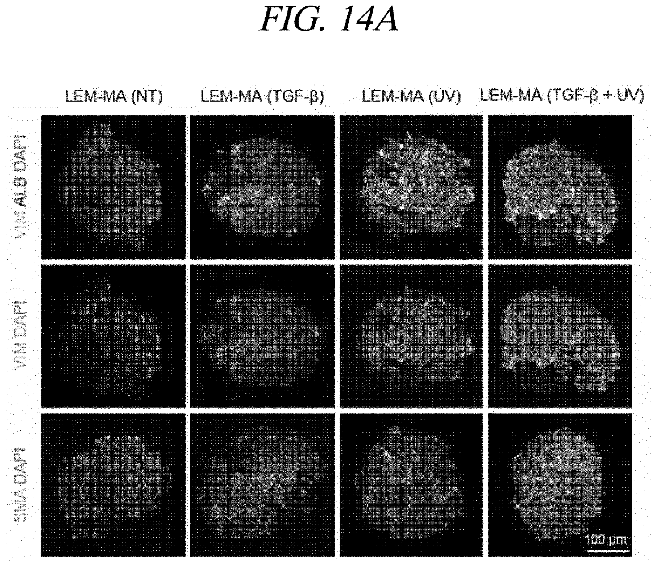

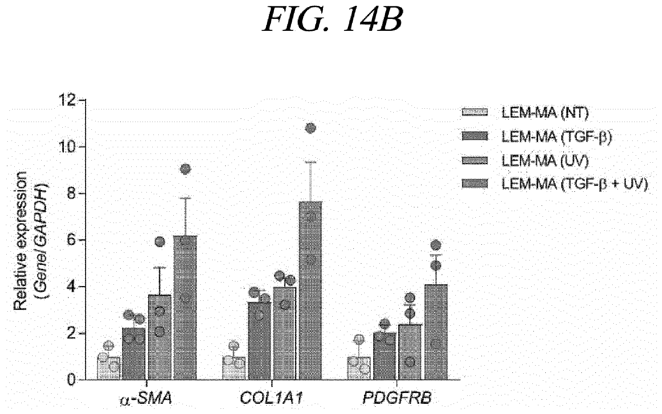

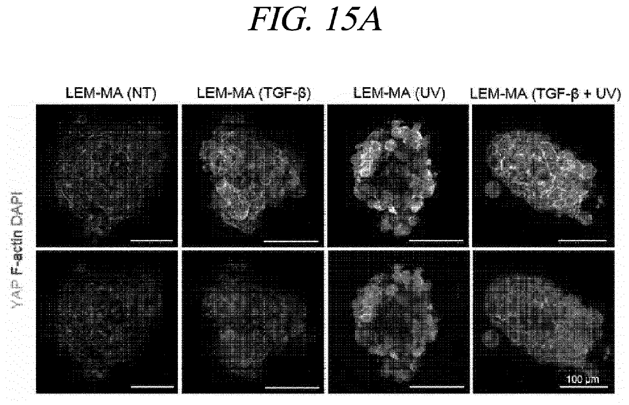

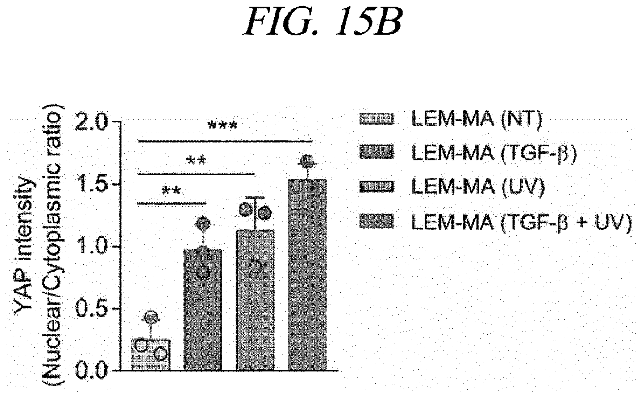

**FIG. 14A** shows the result of analysis of human iPSC-derived liver fibrosis organoids prepared with the ECM-MA hydrogel. **FIG. 14B** shows the result of analysis of human iPSC-derived liver fibrosis organoids prepared with the ECM-MA hydrogel. **FIG. 15A** shows the result of analysis of human iPSC-derived liver fibrosis organoids prepared with the ECM-MA hydrogel. **FIG. 15B** shows the result of analysis of human iPSC-derived liver fibrosis organoids prepared with the ECM-MA hydrogel.

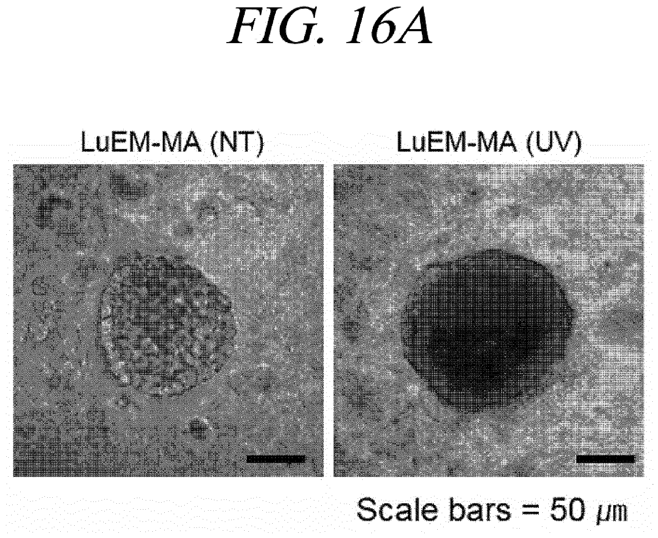

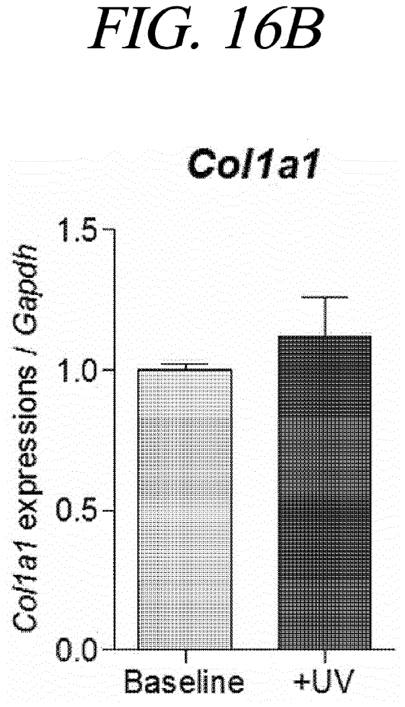

**FIG. 16A** shows the result of modeling fibrosis in lung organoids using the ECM-MA hydrogel. **FIG. 16B** shows the result of modeling fibrosis in lung organoids using the ECM-MA hydrogel.

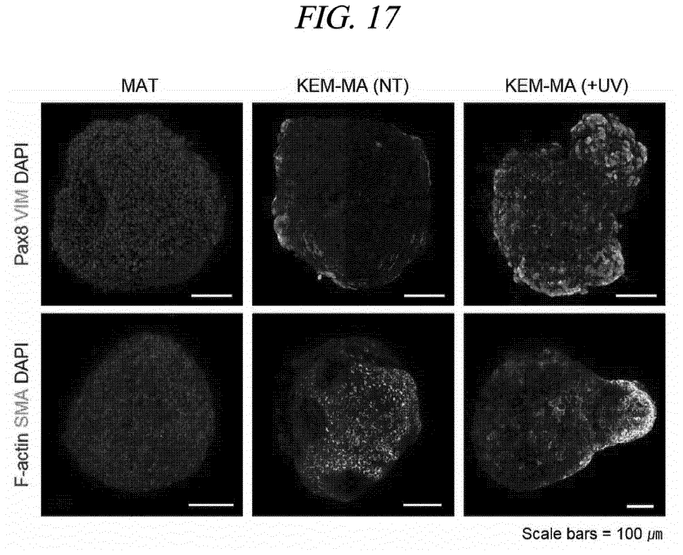

**FIG. 17** shows the result of modeling fibrosis in kidney organoids using the ECM-MA hydrogel.

### BEST MODE FOR CARRYING OUT THE INVENTION

An aspect of the present disclosure provides a composition for hydrogel, including a tissue-derived extracellular matrix modified with an acryl group.

In an embodiment of the present disclosure, the acryl group may be any one or more of an acrylate group, a methacrylate group, and an itaconate group, specifically a methacryl group.

The tissue-derived extracellular matrix is a collection of biopolymers that fill the space inside or outside the tissue and is known as including fibrous proteins such as collagen and elastin, complex proteins such as proteoglycan and glycosaminoglycan, cell adhesion proteins such as fibronectin, laminin, vitronectin, etc. Meanwhile, in the present disclosure, the tissue-derived extracellular matrix can be obtained through decellularization techniques widely known in the art, such as a decellularization solution, and the tissue can include all biological tissues containing an extracellular matrix component. However, the tissue may be any one or more of a liver tissue-derived extracellular matrix, a lung tissue-derived extracellular matrix, a kidney tissue-derived extracellular matrix, a heart tissue-derived extracellular matrix, an intestinal tissue-derived extracellular matrix, a muscle tissue-derived extracellular matrix, a skin tissue-derived extracellular matrix, a pancreatic tissue-derived extracellular matrix, a bone marrow tissue-derived extracellular matrix, a brain tissue-derived extracellular matrix, and a spinal cord tissue-derived extracellular matrix. In an embodiment of the present disclosure, the composition may include a structure represented by the following Chemical Formula 1:

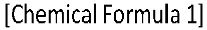

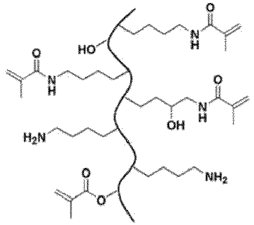

In an embodiment of the present disclosure, the weight ratio of the acryl group and the tissue-derived extracellular matrix may be 1:1 to 8:1, specifically 1:1, 2:1 or 8:1. In examples to be described below, when the acryl group exists as a liquid at room temperature, the ratio of the acryl group and the extracellular matrix means the volume ratio the acryl group to the mass ratio of the extracellular matrix, and the weight ratio depending on the known density of the acryl group is construed as being included in the present disclosure. However, if it is out of the range, the desired effect of the present disclosure cannot be obtained.

As will be described later, the composition undergoes a process of crosslinking to become a hydrogel, and may further include a photoinitiator in order to become a hydrogel through photo-crosslinking. The photoinitiator may be a known material, and in an example of the present disclosure, D2959 may be used as the photoinitiator. In this case, the photoinitiator may be included in the composition in an amount of 0.1% (w/v) to 0.5% (w/v), specifically, 0.3% (w/v).

The modification refers to binding of a specific functional group to a polymer, and in the present disclosure, it means binding of the acryl group to the extracellular matrix. In an embodiment of the present disclosure, the modification may be performed by a treatment at a pH of 8 to 14 for more than 0 hours to less than 8 hours, followed by a treatment at a pH of 6 to 8 for 12 hours to 28 hours. Accordingly, the modification of the tissue-derived extracellular matrix with the acryl group may be performed in a range of 50% to 75%. When the modification with the acryl group is performed out of the range, the desired fibrotic disease cannot be sufficiently simulated.

Another aspect of the present disclosure provides a hydrogel prepared by crosslinking the composition.

In the present disclosure, the term "hydrogel" is a gel containing water as a dispersion medium or basic ingredient. In the present disclosure, the hydrogel includes a tissue-derived extracellular matrix modified with an acryl group.

In an embodiment of the present disclosure, when the hydrogel is prepared, the composition may have a concentration of 0.1% (w/v) to 4.0% (w/v), specifically 0.5% (w/v) to 2.5% (w/v), more specifically 1% (w/v) to 2% (w/v). For example, the composition may have a concentration of 1% (w/v) or 2% (w/v).

The hydrogel may be prepared by a method including a process of preparing a composition for hydrogel by modifying a tissue-derived extracellular matrix with an acryl group, and a process of crosslinking the composition for hydrogel. The crosslinking may include chemical crosslinking caused by UV irradiation, physical crosslinking, or biological crosslinking. Here, the chemical crosslinking by UV irradiation includes photo-crosslinking or crosslinking utilizing a reactive crosslinker. Also, the biological crosslinking includes crosslinking utilizing a binding force between heparin and growth factor or crosslinking using complementary bonding of DNA. Further, the physical crosslinking includes crosslinking by hydrogen bonding, crosslinking by hydrophobic interaction or crosslinking utilizing electrostatic interaction.

In another embodiment of the present disclosure, the composition may be crosslinked through photo-crosslinking to form a hydrogel. In this case, the composition may further include a photoinitiator, and the crosslinking may further include UV irradiation. The photoinitiator may be a known material, and in an example of the present disclosure, D2959 may be used as the photoinitiator. The photoinitiator may be included in the composition in an amount of 0.1% (w/v) to 0.5% (w/v), specifically, 0.3% (w/v). Further, the UV irradiation may be performed under known conditions. Specifically, the UV irradiation may be performed for 1 minute to 20 minutes. In the present disclosure, for example, a hydrogel may be formed through photo-crosslinking by irradiating a light source of 365 nm and 8900 µW/cm² for 1 minute, 3 minutes or 10 minutes.

Yet another aspect of the present disclosure provides a method of preparing a composition for hydrogel, including a process of modifying a tissue-derived extracellular matrix with an acryl group.

As described above, the weight ratio of the tissue-derived extracellular matrix and the acryl group may be 1:1 to 1:8, specifically 1:1, 2:1, or 8:1.

Further, the composition may further include a photoinitiator as described above. In an embodiment, 0.3% (w/v) of D2959 as a photoinitiator may be included in the composition.

The modification may be performed by a treatment at a pH of 8 to 14 for more than 0 hours to less than 8 hours, followed by a treatment at a pH of 6 to 8 for 12 hours to 28 hours. Accordingly, the modification of the tissue-derived extracellular matrix with the acryl group may be performed in a range of 50% to 75%.

As described above, in order for the hydrogel to exhibit the desired properties, the composition may have a concentration of 0.1% (w/v) to 4.0% (w/v), specifically 0.5% (w/v) to 2.5% (w/v), more specifically 1% (w/v) to 2% (w/v). For example, the composition may have a concentration of 1% (w/v) or 2% (w/v).

Still another aspect of the present disclosure provides a method of preparing a hydrogel, including: a process of preparing a composition for hydrogel by modifying a tissue-derived extracellular matrix with an acryl group; and a process of crosslinking the composition for hydrogel.

The process of preparing a composition for hydrogel is performed to prepare the composition for hydrogel by modifying the above-described tissue-derived extracellular matrix with an acryl group.

The process of crosslinking is performed to prepare a hydrogel by crosslinking the composition for hydrogel.

When process of crosslinking is performed by photo-crosslinking, the composition for hydrogel may further include a photoinitiator, and the crosslinking may further include UV irradiation.

### MODE FOR CARRYING OUT THE INVENTION

Hereinafter, one or more specific embodiments will be described in more detail through examples. However, these examples are for illustrative purposes of one or more embodiments, and the scope of the present disclosure is not limited to these examples.

### Example 1: Preparation and characterization of ECM-MA derivative

### Example 1-1. Preparation and schematic diagram of ECM-MA derivative

An extracellular matrix (ECM) was obtained by subjecting a living tissue to appropriate decellularization and solutionization, and then modified with a methacryl (MA) group in a chemical way by treating a substance (e.g., methacrylic anhydride; MAA) containing the MA group and capable of binding to a functional group in the ECM to synthesize a methacrylated tissue-derived extracellular matrix (ECM-MA).

Specifically, an ECM solution was prepared at a concentration of 2 mg/ml, and the MAA was treated in various ratios (ECM mass: MAA volume = 1:1, 1:2, 1:8 mg/pd ratio), followed by a reaction at 4°C for 24 hours (the reaction was carried out by varying the time for which the pH was maintained at 8 or higher in 24 hours), and then, the unreacted material was removed by dialysis to prepare an ECM-MA derivative (**FIG. 1**). It was confirmed that the proportion of the modified MA group was about 55% to about 75%.

### Example 1-2. Fabrication of hydrogel using ECM-MA derivative and confirmation of controllability of physical properties depending on photo-crosslinking

It was confirmed that the ECM-MA derivative synthesized in Example 1-1 (liver extracellular matrix (LEM) which is a decellularized liver tissue-derived ECM; LEM-MA) can form a hydrogel through self-assembly of collagen and major components of the ECM under physiological conditions (1×PBS, neutral pH, 37°C) similar to the in vivo environment (please see a reference character "a" in **FIG**. **2**).

Herein, it was confirmed that the mechanical strength of the hydrogel can be further increased by inducing additional crosslinking of the MA group modified on the ECM polymer through photo-polymerization (please see a reference character "b" and "c" in FIG. 2) [The photo-polymerization was induced by using D2959 (final concentration: 0.3% (w/v)) as a photoinitiator and irradiating UV light for 10 minutes with a UV light source (365 nm, 8900 µW/cm²)]. In particular, it was confirmed that the mechanical strength of the LEM-MA hydrogel can be easily controlled by controlling the amount of light energy during photo-crosslinking (herein, by controlling the light treatment time) (please see a reference character "d" in **FIG. 2**). It was confirmed that the physical properties of LEM-MA increased in proportion to the increase of the photo-crosslinking time for which UV was irradiated.

### Example 1-3. Confirmation of controllability of mechanical properties by adjusting modification ratio of MA group in ECM-MA derivative

The proportion of a compound containing an ECM and an MA group (herein, methacrylic anhydride; MAA) was adjusted when methacrylate-modified extracellular matrix (ECM-MA, specifically, LEM which is a decellularized liver tissue-derived ECM; LEM-MA) was synthesized, which confirmed that increments of mechanical properties during hydrogel formation through photo-crosslinking can be controlled.

Specifically, the LEM-MA hydrogel had a final concentration of 2% (w/v), and photo-crosslinking was induced by using D2959 (final concentration: 0.3% (w/v)) as a photoinitiator and irradiating UV light for 10 minutes with a UV light source (365 nm, 8900 µW/cm²) (1X LEM-MA in **FIGS. 3A****,** **3B** and **3C** was synthesized at LEM:MAA=1:2 (w/v) and 4X LEM-MA in **FIGS. 3D****,** **3E** and **3F** was synthesized at LEM:MAA=1:8 (w/v)).

As for the mechanical properties, a storage modulus G' and loss modulus G" were measured in a frequency sweep mode using a rheometer. Elastic modulus was obtained by calculating the average value of G' values measured at 1 Hz and used to compare the mechanical properties, and elasticity was obtained by calculating the ratio of G" and G' (G"/G') measured values at 1 Hz and used to compare strength.

As a result, as shown in **FIGS. 3A****,** **3B****,** **3C****,** **3D****,** **3E** and **3F****,** in the case of 1X LEM-MA hydrogel, the elastic modulus of a 10-minute UV treatment group (+UV group) increased by 3.47 times from 115.64 Pa to 401.5 Pa compared to a non-UV group (-UV group), and the elasticity decreased from 0.290 to 0.211. In the case of 4X LEM-MA hydrogel, the elastic modulus increased by 5.26 times from 207.092 Pa in the non-UV group (-UV group) to 1088.42 Pa in the UV treatment (+UV group), and the elasticity decreased from 0.283 to 0.176.

This implies that it is possible to control the maximum mechanical properties of the LEM-MA hydrogel by synthesizing LEM-MA derivatives at various ratios of LEM:MAA during synthesis and also possible to control physical properties and ratio of elasticity, which can be changed through photo-crosslinking, compared to a baseline hydrogel.

### Test Example 1: Characterization of ECM-MA hydrogel

### Test Example 1-1. Confirmation of possibility of culturing mouse adult stem cell-derived liver organoid using ECM-MA hydrogel (1)

In order to confirm the possibility of culturing liver organoids using the LEM-MA derivative-based hydrogel prepared in Examples 1-2, liver organoids were cultured in Matrigel (MAT), which is a conventional culture scaffold, and LEM hydrogels, which are decellularized liver tissue-derived scaffolds without being modified with methacrylic anhydride (MAA), and then compared to each other. Specifically, liver organoids were prepared using bile duct cells isolated from mouse liver tissue and cultured in MAT for 10 days and then sub-cultured in LEM and LEM-MA scaffolds.

As a result, it can be seen from **FIG. 4** that liver organoids were well cultured in LEM (concentration: 6 mg/ml) and LEM-MA hydrogel [in this case, crosslinking, not photo-crosslinking, was induced through self-assembly of ECM fibers under physiological conditions (1X PBS, neutral pH, 37°C)] maintaining their morphology as in Matrigel (MAT). The LEM hydrogel, a decellularized liver tissue-derived scaffold, was applied at a concentration of 6 mg/ml at which physical properties are most similar to those of Matrigel and a liver organoid is well formed, as confirmed through previous studies.

### Test Example 1-2. Confirmation of possibility of culturing mouse adult stem cell-derived liver organoid using ECM-MA hydrogel (2)

Immunostaining of liver organoids cultured in Matrigel (MAT), which is most widely used for organoid culture, a decellularized liver tissue-derived LEM hydrogel (6 mg/ml), and the prepared LEM-MA hydrogel (crosslinking, not photo-crosslinking, through self-assembly of ECM fibers at 37°C) was performed. Specifically, liver organoids were prepared using bile duct cells extracted from mouse liver tissue, cultured in Matrigel for 10 days, sub-cultured in LEM and LEM-MA scaffolds and then compared to each other through immunostaining on day 7 of culture. **(****FIG. 5A**).

When compared to the liver organoids cultured in MAT and LEM hydrogels as positive controls by immunostaining for KRT19, a liver tissue-specific marker, the liver organoids cultured in LEM-MA hydrogels well expressed the liver tissue-specific marker at similar levels to that of the positive controls, as shown in **FIG. 5A****.**

Further, when the gene expression levels of the liver organoids cultured in MAT, LEM and LEM-MA hydrogels were compared by quantitative PCR (qPCR), the expression of stem ness-related markers was slightly decreased in LEM and LEM-MA 2% and maintained similarly in MAT and LEM-MA 1% (**FIG. 5B**).

It was confirmed that the expression of hepatic differentiation-related markers (KRT19, HNF4A, HES1 and KRT18) was observed similarly in MAT, LEM and LEM-MA, and the expression of apoptosis-related markers (CASP3 and BAX) was slightly lower in the organoids cultured in LEM and LEM-MA than in the organoid cultured in MAT **(****FIG. 5C**).

Overall, it was verified that like MAT and LEM, which are conventional organoid culture matrices, the LEM-MA hydrogel can also be applied for liver organoid culture.

### Test Example 1-3. Modeling of fibrosis in mouse adult stem cell-derived liver organoid using ECM-MA hydrogel (1)

When a liver organoid was cultured in the LEM-MA hydrogel scaffold of Example 1-2, photo-crosslinking of LEM-MA was further induced to model liver fibrosis (final LEM-MA concentration: 2% (w/v), photoinitiator D2959 concentration: 0.3% (w/v)). Here, the mechanical strength of the hydrogel was increased by adjusting the time of irradiation with a UV light source (365 nm, 8900 µW/cm²). The gene expression level of the liver organoid was analyzed while varying the time of photo-crosslinking with UV from 1 minute to 3 minutes and 10 minutes. The liver organoid was cultured in Matrigel for 10 days, sub-cultured in the LEM-MA scaffold and treated with UV. Then, the gene expression levels were compared on day 3 of culture **(****FIGS. 6A** and **6B****).**

When the gene expression levels of the liver organoids depending on the UV photo-crosslinking time were compared by quantitative PCR (qPCR), the expression of a liver fibrosis-related marker (α-SMA) and the expression of a stem ness-related marker were gradually increased as the UV treatment time for photo-crosslinking was increased. The expression of a differentiation-related marker (HES1) was slightly decreased as the photo-crosslinking time was increased. Also, the expression of an apoptosis marker (BAX) was maintained without a significant difference depending on the photo-crosslinking time **(****FIG. 6B**).

Accordingly, it was confirmed that fibrosis was best induced in the case of treatment with UV for 10 minutes and liver stem cells were activated without a significant effect on apoptosis. Thus, this condition was confirmed as the most effective crosslinking time to induce a fibrosis model.

### Test Example 1-4. Modeling of fibrosis in mouse adult stem cell-derived liver organoid using ECM-MA hydrogel (2)

In order to construct a more precise liver fibrosis model, a method of biochemically inducing fibrosis using a fibrosis-inducing cytokine (TGF-β), a method of inducing fibrosis by increasing the mechanical properties through UV photo-crosslinking (light source: 365 nm, 8900 µW/cm², UV irradiation for 10 minutes, photoinitiator D2959 concentration: 0.3% (w/v)), and a method of inducing fibrosis by simultaneously applying biochemical and mechanical stimulation were compared (LEM-MA hydrogel concentration: 2% (w/v)).

Specifically, when immunostaining of liver organoids cultured for 3 days was performed to compare the degrees of induction of liver fibrosis in the respective groups, fibrosis markers (SMA and VIM) were hardly expressed in the MAT and LEM-MA groups, but the expression levels of the fibrosis markers were increased in the group in which fibrosis was biochemically induced through a treatment with TGF-β (10 ng/ml) and the group in which the mechanical properties were increased through UV photo-crosslinking. It was confirmed that the fibrosis markers were best expressed in the group in which fibrosis was induced by a treatment with two types of simulation **(****FIG. 7A**).

Similarly, when the degree of fibrosis in each group was checked through gene expression comparison by performing a quantitative PCR analysis on day 3 of culture, fibrosis-related genes (α-SMA and COL1A2) were increased in the LEM-MA (NT), LEM-MA (TGF-β) and LEM-MA (UV) groups, compared to the MAT group and most significantly increased in the LEM-MA (TGF-β+UV) group **(****FIG. 7B**). The expression level of an inflammation-related gene (TNF-α) was increased in the LEM-MA (NT) and LEM-MA (UV) groups compared to the MAT group, and most significantly increased in the LEM-MA (TGF-β) group and LEM-MA (TGF-β+UV) groups treated with a TGF-β factor (**FIG. 7B**).

Accordingly, it was confirmed that an organoid model that best implements the real liver fibrosis environment can be constructed by combination of the biochemical induction using fibrotic cytokines and the increase in mechanical properties through UV photo-crosslinking.

### Test Example 2: Optimization of ECM-MA synthesis conditions for modeling fibrosis in human iPSC-derived liver organoid

### Test Example 2-1. Optimization of ECM-MA synthesis conditions for modeling fibrosis in human iPSC-derived liver organoid

In order to verify the applicability of the ECM-MA hydrogel of the present disclosure to human organoids and screen for ECM-MA conditions that best induce fibrosis, human induced pluripotent stem cell(hiPSC)-derived liver organoids were cultured using LEM-MA synthesized under different conditions **(****FIG.** 8A).

Herein, iPSC-derived liver organoids were prepared by co-culturing liver cells and human umbilical vein endothelial cells differentiated from iPSC through endoderm and hepatic endoderm stages, human mesenchymal stem cells, and hepatic stellate cells (differentiated from iPSC essential for liver fibrosis through mesoderm and mesothelial cell stages) at a ratio of 10:7:2:2 in ultra low attachment plates (U-bottom plates).

Hydrogels (LEM-MA concentration: 2% (w/v)) were fabricated using LEM-MA prepared by varying the proportion of methacrylic anhydride (MAA) and the reaction-accelerating pH (> 8) retention time in a total reaction time of 24 hours as described below, and their performance was evaluated:
#1: LEM:MAA = 1:2 (mg/µl), 3 hours of pH maintenance (> 8) + 21 hours thereafter - synthesized by a reaction at 4°C for a total of 24 hours.

In the case of group #1, it was difficult to form a stable hydrogel only by crosslinking through self-assembly of ECM fibers, and, thus, photo-crosslinking was induced by UV irradiation for 1 minute (photoinitiator D2959 concentration: 0.3% (w/v), UV light source: 365 nm, 8900 µW/cm²) to prepare a baseline hydrogel.

#2: LEM:MAA = 1:2 (mg/µl), 6 hours of pH maintenance (> 8) + 18 hours thereafter - synthesized by a reaction at 4°C for a total of 24 hours.

In the case of group #2, it was difficult to form a stable hydrogel only by crosslinking through self-assembly of ECM fibers, and, thus, photo-crosslinking was induced by UV irradiation for 1 minute (photoinitiator D2959 concentration: 0.3% (w/v), UV light source: 365 nm, 8900 µW/cm²) to prepare a baseline hydrogel.

#3: LEM:MAA = 1:2 (mg/µl), 0 hours of pH maintenance (> 8) + 24 hours thereafter - synthesized by a reaction at 4°C for a total of 24 hours.

In the case of group #3, a baseline hydrogel was prepared by inducing crosslinking through self-assembly of ECM fibers under physiological conditions (1X PBS, neutral pH, 37°C) without UV irradiation.

#4: LEM:MAA = 1:1 (mg/µl), 3 hours of pH maintenance (> 8) + 21 hours thereafter - synthesized by a reaction at 4°C for a total of 24 hours.

In the case of group #4, a baseline hydrogel was prepared by inducing crosslinking through self-assembly of ECM fibers under physiological conditions (1X PBS, neutral pH, 37°C) without UV irradiation.

Human iPSC-derived liver organoids were cultured for 5 days in MAT, which is a conventional culture scaffold, LEM, which was not modified with MAA, and LEM-MA hydrogels synthesized under four conditions, respectively, and the gene expression levels of hepatic differentiation markers (AFP, HNF4A and ALB) were compared (**FIG. 8B**). As a result, it was confirmed that the expression levels of hepatic differentiation markers in the liver organoid cultured in the hydrogel under the LEM-MA #1 condition were similar to or higher than those of MAT and LEM, and the expression levels of hepatic differentiation markers in the hydrogels under the LEM-MA #2 and #4 conditions were significantly decreased. Also, it was confirmed that the expression levels in the hydrogel of LEM-MA #3 condition were similar to or somewhat lower than those of the MAT group.

Accordingly, it was confirmed that the derivatives synthesized under the LEM-MA #2 and #4 conditions are not suitable for modeling fibrosis in human liver organoids.

### Test Example 2-2. Optimization of ECM-MA conditions for modeling fibrosis in human iPSC-derived liver organoid

After the LEM-MA hydrogels (2% (w/v) concentration) synthesized under four different conditions in Test Example 2-1 were subjected to UV treatment for 10 minutes, the physical properties of the hydrogels were increased through photo-crosslinking (photoinitiator D2959 concentration: 0.3 % (w/v), UV light source: 365 nm, 8900 µW/cm²), followed by a comparison in terms of the efficiency in induction of fibrosis in human iPSC-derived liver organoids (+UV group). When the liver organoids were cultured for 5 days and then compared to the liver organoids cultured in the LEM-MA hydrogels in a baseline state (baseline group) in terms of the gene expression levels of fibrosis markers (α-SMA and VIM) through a quantitative PCR analysis, it was confirmed that the expression levels of fibrosis markers were most significantly increased in the liver organoid cultured in the LEM-MA #1 hydrogel photo-crosslinked with UV compared to the liver organoids cultured in the LEM-MA hydrogels photo-crosslinked with UV under the other synthesis conditions **(****FIGS. 9A** and **9B****).**

Together with the previous results, it was confirmed that the LEM-MA hydrogels synthesized under the LEM-MA #1 and #3 conditions were suitable for organoid differentiation and viability maintenance, but the LEM-MA hydrogel synthesized under the LEM-MA #1 condition showed a significantly higher degree of induction of fibrosis than the LEM-MA hydrogel synthesized under the LEM-MA #3 condition, and, thus, the LEM-MA synthesized under the LEM-MA #1 condition was most suitable for modeling fibrosis in human iPSC-derived liver organoids.

### Test Example 2-3. Optimization of ECM-MA conditions for modeling fibrosis in human iPSC-derived liver organoid (checking the degree of modification depending on synthetic condition)

It was confirmed from the organoid culture test of Test Example 2-2 that the hydrogel synthesized under the LEM-MA #2 and #4 conditions is not suitable for modeling fibrosis in human liver organoids, and the LEM-MA #1 condition of the LEM-MA #1 and #3 conditions is more suitable for the present study. In order to determine the cause, an analysis for identifying the degree of methacrylation on the LEM was conducted.

Specifically, to this end, 2,4,6-trinitrobenzene sulfonic acid (TNBSA) having reactivity to a primary amine group was used to quantify the degree of methacrylation (a decrement after modification as a result of comparison to the amount of amine group present in the LEM before a chemical reaction for methacryl group modification = degree of methacrylation) by measuring a decrement of an amine group caused by the methacryl group modification in the LEM.

**Accordingly,** as shown in **FIG. 10**, it was confirmed that the degree of methacrylation increases as the pH (> 8) retention time in which a chemical reaction is accelerated increases. Therefore, in the case of LEM-MA #2 condition (compared to the LEM-MA #1 and #3 conditions), excessive methacrylation occurred and functional groups of ECM proteins present in the LEM were consumed. Accordingly, it can be speculated that tissue-specific factors such as ECM proteins/peptides necessary for liver organoid growth and differentiation do not function properly.

### Test Example 2-4. Optimization of ECM-MA conditions for modeling fibrosis in human iPSC-derived liver organoid (difference in physical properties depending on the degree of modification)

In the evaluation of the above-described culture ability and the ability to induce fibrosis through photo-crosslinking (photoinitiator D2959 concentration: 0.3% (w/v), UV light source: 365 nm, 8900 µW/cm²), the LEM-MA hydrogels synthesized under the LEM-MA #1 condition (degree of methacrylation: 68.38%) and the LEM-MA #3 condition (degree of methacrylation: 59.12%) functioned well. Particularly, the hydrogel synthesized under the LEM-MA #1 condition showed an increase in physical properties (elastic modulus) by about up to 8.85 times after photo-crosslinking (UV treatment for 10 minutes) compared to the hydrogel in a baseline state, and the hydrogel synthesized under the LEM-MA #3 condition showed a much smaller increase in physical properties by about 3.47 times after a UV treatment for 10 minutes compared to the hydrogel in a baseline state. This was confirmed through rheological measurement using a rheometer.

Also, when a UV treatment was performed for 10 minutes, the elastic modulus of the hydrogel was about 1163.28 Pa for the LEM-MA #1 condition, while it was 401.5 Pa for LEM-MA #3 condition, which confirmed that there is a difference by about 2.9 times in the value of mechanical properties induced during a UV treatment for the same period of time (**FIGS**. **11A** and **11B****).**

Accordingly, it is assumed that the reason why modeling of fibrosis is more efficient in the hydrogel synthesized under the LEM-MA #1 condition is because an increase in mechanical strength is more advantageous for activation of biophysical signals related to fibrosis induction. Therefore, the following fibrosis modeling test was conducted using the LEM-MA hydrogel synthesized under the LEM-MA #1 condition.

### Test Example 2-5. Optimization of ECM-MA conditions for modeling fibrosis in human iPSC-derived liver organoid (difference in internal structure depending on the degree of modification)

The internal structures of the LEM-MA hydrogels synthesized under the LEM-MA #1 and #3 conditions, respectively, were observed using a scanning electron microscope (SEM) to determine the cause of the difference in mechanical strength of the LEM-MA hydrogels (photo-crosslinking conditions-photoinitiator D2959 concentration: 0.3% (w/v), UV light source: 365 nm, 8900 µW/cm², 10-minute irradiation).

As for the LEM-MA hydrogels prepared under the LEM-MA #1 condition, it was confirmed that the photo-crosslinking group (treated with UV for 10 minutes) was turned to have an overall denser internal structure after curing through UV crosslinking than the baseline group at 500X magnification (FIG. **12A**). Accordingly, it was confirmed that the physical properties increased as an internal polymer network became stronger in the process of curing through photo-crosslinking. Further, referring to the 20000X magnification images, a unique nanofiber structure of the ECM hydrogel is observed. Therefore, it is assumed that the mechanical properties were enhanced because a dense bundle structure was formed through additional crosslinking between ECM nanofibers due to photo-crosslinking.

**As** for the LEM-MA hydrogels prepared under the LEM-MA #3 condition, network structures composed of thin nanofibers, which can be observed in the conventional ECM hydrogel, were observed in both the baseline group and the photo-crosslinking group (treated with UV for 10 minutes) at 500X magnification and 20000X magnification in contrast to the LEM-MA hydrogels prepared under the LEM-MA #1 condition. This is assumed to be because the degree of methacrylation was lower in the LEM-MA hydrogels prepared under the LEM-MA #3 condition than in the LEM-MA hydrogels prepared under the LEM-MA #1 condition, and, thus, self-assembly of collagen fibers occurred faster and more easily in the LEM-MA hydrogels prepared under the LEM-MA #3 condition than in the LEM-MA hydrogels prepared under the LEM-MA #1 condition **(****FIG. 12B****).** Also, although the internal structure network became slightly denser after curing through UV photo-crosslinking, there was no significant difference between the baseline group and the photo-crosslinking group (treated with UV for 10 minutes). Therefore, it is assumed that an increment of mechanical properties caused by photo-crosslinking was small in the hydrogels synthesized under the LEM-MA #3 condition.

### Test Example 3. Evaluation of organoid cultured in ECM-MA hydrogel

### Test Example 3-1. Evaluation of toxicity of human iPSC-derived liver organoid depending on photo-crosslinking treatment time based on ECM-MA hydrogel

In order to check whether apoptosis and cytotoxicity do not appear due to UV during photo-crosslinking (photoinitiator D2959 concentration: 0.3% (w/v), UV light source: 365 nm, 8900 µW/cm²) of the prepared LEM-MA hydrogel (synthesis condition #1, LEM-MA concentration: 2% (w/v)), human iPSC-derived liver organoids were subjected to UV treatment while varying the UV photo-crosslinking time from 1 minute to 3 minutes and 10 minutes. Then, the cytotoxicity of the organoids was evaluated.

Specifically, it was confirmed that when the prepared human iPSC-derived liver organoids were cultured in the LEM-MA hydrogels and treated with UV for each hour, the liver organoids in the group treated for 10 minutes were well cultured. Also, it was confirmed that even when cell viability was checked through Live/Dead staining after 3 days of culture, the cells were well alive in the organoids. (FIG. 13A).

It was confirmed that when the protein expression in liver organoids depending on the UV photo-crosslinking time was compared by immunostaining, F-actin, a filament fiber marker, and AFP, SOX17 and ALB, hepatic differentiation markers, were well expressed in the organoids even after a treatment with UV for 10 minutes. Accordingly, it was confirmed that the 10-minute UV treatment does not cause toxicity in the organoids, but causes an increase in mechanical properties of the LEM-MA hydrogels **(****FIG. 13B****).**

### Test Example 3-2. Analysis of human iPSC-derived liver fibrosis organoid fabricated based on ECM-MA hydrogel (1)

In order to construct a precise liver fibrosis model, a method of biochemically inducing fibrosis using a fibrosis-inducing cytokine (TGF-β), a method of inducing fibrosis by increasing the mechanical properties through UV photo-crosslinking (photoinitiator D2959 concentration: 0.3% (w/v), UV light source: 365 nm, 8900 µW/cm², UV irradiation for 10 minutes), and a method of inducing fibrosis by simultaneously applying biochemical and mechanical stimulation were compared in the human iPSC-derived liver organoids, and the group in which fibrosis was most likely to occur was identified. Specifically, the LEM-MA of the group #1 was used and the concentration was 2% (w/v).

When immunostaining of liver organoids was performed on day 5 of culture to compare the degrees of induction of liver fibrosis in the respective groups, fibrosis markers (SMA and VIM) were hardly expressed in the LEM-MA (NT) group, but the expression levels of the fibrosis markers were increased in the group in which fibrosis was biochemically induced through a treatment with 10 ng/ml TGF-β and the group in which the mechanical properties were increased through UV photo-crosslinking, as shown in **FIG. 14A****.** It was confirmed that the fibrosis markers were best expressed in the group in which fibrosis was induced by a treatment with two types of simulation. Further, the expression of ALB, a hepatic differentiation marker was decreased in the groups in which fibrosis was induced, compared to the no-treatment (NT) group without any treatment, which confirmed that functionality of liver was decreased.

When the degree of fibrosis in each group was checked through gene expression comparison by performing a quantitative PCR analysis, fibrosis-related genes (α-SMA, COL1A2 and PDGFRB) were increased in the LEM-MA+TGF-β and LEM-MA+UV groups, compared to the LEM-MA (NT) group and most significantly increased in the LEM-MA+TGF-β+UV group, as shown in **FIG. 14B****.**

Accordingly**,** it was confirmed that a human iPSC-derived liver fibrosis organoid model that best implements the real liver fibrosis environment can be constructed by combination of the biochemical induction using fibrotic cytokines and the increase in mechanical properties through UV photo-crosslinking.

### Test Example 3-3. Analysis of human iPSC-derived liver fibrosis organoid fabricated based on ECM-MA hydrogel (2)

Yes-associated protein (YAP) is an important protein in a signal transduction pathway that performs an important function for cell proliferation, apoptosis and migration, and is known to be activated and highly expressed in patients with liver fibrosis or cirrhosis. In order to check whether the expression of the YAP protein is also increased in the liver organoid fibrosis model prepared through UV photo-crosslinking (photoinitiator D2959 concentration: 0.3% (w/v), UV light source: 365 nm, 8900 µW/cm², UV irradiation for 10 minutes) based on the LEM-MA hydrogel (group # 1, LEM-MA concentration: 2% (w/v)), immunostaining and a quantitative analysis were performed on day 5 of culture.

Accordingly**,** as shown in **FIG. 15A****,** the expression of the YAP protein was hardly observed in the no treatment (NT) group in which any treatment was not given to the liver organoids, whereas the expression of the YAP protein increased in the group in which fibrosis was biochemically induced through a treatment with 10 ng/ml TGF-β and the group in which the mechanical properties were increased through UV photo-crosslinking. It was confirmed that the YAP protein was best expressed in the group in which fibrosis was induced by a treatment with two types of simulation.

The YAP protein is present in both the nucleus and the cytoplasm depending on the cell condition. It is known that as the severity of fibrosis increases, remodeling of the surrounding ECM occurs and the YAP protein is mainly present in the nucleus due to nuclear localization. It was confirmed that even when it was calculated and quantified as the ratio of the amount of the YAP protein expressed in the nucleus to the amount of the YAP protein expressed in the cytoplasm in order to analyze the activity of the YAP protein, the liver organoid in which fibrosis was induced by a treatment with a biochemical factor and physical crosslinking exhibited the highest activity compared to the NT group (**FIG. 15B**).

Accordingly, it was confirmed that the liver organoid fibrosis model fabricated through UV photo-crosslinking based on the LEM-MA hydrogel can provide a more precise fibrosis in vitro model that can implement fibrosis signal transduction at a molecular level with increased YAP pathway activity as in the actual fibrotic tissue.

### Test Example 4: Modeling of fibrosis in lung organoid using ECM-MA hydrogel

Fibrosis was induced in a lung organoid by increasing the mechanical properties of a hydrogel through UV photo-crosslinking (light source: 365 nm, 8900 µW/cm², UV irradiation for 10 minutes, photoinitiator D2959 concentration: 0.3% (w/v)) of an LuEM-MA derivative prepared by modifying a decellularized lung tissue-derived extracellular matrix (LuEM) with MA for lung fibrosis modeling (LuEM-MA hydrogel concentration: 2% (w/v)).

When the mechanical properties of the LuEM-MA hydrogels were enhanced through UV photo-crosslinking, fibrosis was induced in the cultured lung organoids **(****FIG. 16A****).**

It was confirmed that when a quantitative PCR analysis was performed to lung organoids cultured for 7 days to quantitatively compare the degree of induction of fibrosis between the LuEM-MA (NT) group and the LuEM-MA (+UV) group, the expression level of Col1a1, a fibrosis marker, was higher in the LuEM-MA (UV) group than in the LuEM-MA (NT) group **(**FIG. **16B****).**

**Therefore,** it was confirmed that a lung organoid-based fibrotic disease model can be fabricated by increasing the mechanical properties of the LuEM-MA hydrogel through UV photo-crosslinking.

### Test Example 5: Modeling of fibrosis in kidney organoid using ECM-MA hydrogel

Fibrosis was induced by increasing the mechanical properties of a hydrogel through UV photo-crosslinking (light source: 365 nm, 8900 µW/cm², UV irradiation for 10 minutes, photoinitiator D2959 concentration: 0.3% (w/v)) of a KEM-MA derivative prepared by modifying a decellularized kidney tissue-derived extracellular matrix (KEM) with MA to fabricate a kidney fibrosis model (KEM-MA hydrogel concentration: 2% (w/v)).

Immunostaining of kidney organoids cultured for 7 days was performed to compare the degrees of induction of fibrosis in the respective groups, SMA and VIM, fibrosis-related markers, were hardly expressed in the MAT (Matrigel) group and the KEM-MA (NT) group, but the expression of the SMA and VIM was significantly increased in the group (KEM-MA(+UV)) in which the mechanical properties of the hydrogel were increased through UV photo-crosslinking (FIG. 17).

Therefore, it was confirmed that a kidney organoid-based fibrotic disease model can be fabricated by increasing the mechanical properties of the KEM-MA hydrogel through UV photo-crosslinking.

The present disclosure has been described with reference to the preferred exemplary embodiments thereof. It can be understood by a person with ordinary skill in the art that the present disclosure can be implemented as being modified and changed within the scope departing from the spirit and the scope of the present disclosure. Accordingly, the above-described exemplary embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. A composition for hydrogel, comprising:
a tissue-derived extracellular matrix modified with an acryl group.

2. The composition for hydrogel of Claim 1,
wherein the acryl group is any one or more of an acrylate group, a methacrylate group, and an itaconate group.

3. The composition for hydrogel of Claim 1,
wherein the tissue-derived extracellular matrix is any one or more of a liver tissue-derived extracellular matrix, a lung tissue-derived extracellular matrix, a kidney tissue-derived extracellular matrix, a heart tissue-derived extracellular matrix, an intestinal tissue-derived extracellular matrix, a muscle tissue-derived extracellular matrix, a skin tissue-derived extracellular matrix, a pancreatic tissue-derived extracellular matrix, a bone marrow tissue-derived extracellular matrix, a brain tissue-derived extracellular matrix, and a spinal cord tissue-derived extracellular matrix.

4. The composition for hydrogel of Claim 1,
wherein the weight ratio of the acryl group and the tissue-derived extracellular matrix is 1:1 to 8:1.

5. The composition for hydrogel of Claim 1,
wherein the modification is performed by a treatment at a pH of 8 to 14 for more than 0 hours to less than 8 hours, followed by a treatment at a pH of 6 to 8 for 12 hours to 28 hours.

6. The composition for hydrogel of Claim 1,
wherein the modification of the tissue-derived extracellular matrix with the acryl group is performed in a range of 50% to 75%.

7. A hydrogel prepared by crosslinking the composition of Claim 1.

8. The hydrogel of Claim 7,
wherein the composition has a concentration of 0.1% (w/v) to 4.0% (w/v).

9. The hydrogel of Claim 7,
wherein the composition further includes a photoinitiator, and
the crosslinking further includes UV irradiation.

10. The hydrogel of Claim 9,
wherein the UV irradiation is performed for 1 minute to 20 minutes.

11. A method of preparing a composition for hydrogel, comprising:
a process of modifying a tissue-derived extracellular matrix with an acryl group.

12. The method of preparing a composition for hydrogel of Claim 11,
wherein the weight ratio of the tissue-derived extracellular matrix and the acryl group is 1:1 to 1:8.

13. The method of preparing a composition for hydrogel of Claim 11,
wherein the modification is performed by a treatment at a pH of 8 to 14 for more than 0 hours to less than 8 hours, followed by a treatment at a pH of 6 to 8 for 12 hours to 28 hours.

14. A method of preparing a hydrogel, comprising:
a process of preparing a composition for hydrogel by modifying a tissue-derived extracellular matrix with an acryl group; and
a process of crosslinking the composition for hydrogel.

15. The method of preparing a hydrogel of Claim 14,
wherein the composition for hydrogel further includes a photoinitiator, and
the crosslinking further includes UV irradiation.
